(19) Europäisches Patentamt
European Patent Office
Office européen des brevets

(11) EP 2 420 510 B1

(12) **EUROPEAN PATENT SPECIFICATION**

(45) Date of publication and mention of the grant of the patent:
**01.05.2013 Bulletin 2013/18**

(51) Int Cl.:
***C07K 14/665*** *(2006.01)* ***C07K 14/70*** *(2006.01)*
***A61K 8/64*** *(2006.01)* ***A61Q 19/08*** *(2006.01)*

(21) Application number: **11177637.3**

(22) Date of filing: **16.08.2011**

(54) **Nicotinoyl peptide derivatives and cosmetic composition comprising the same**

Nicotinoyl-Peptidkonjugate und sie enthaltende kosmetische Zusammensetzungen

Conjugués de peptides avec un résidu nicotinoyle et compositions cosmétiques les comprenant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2010 KR 20100080687**

(43) Date of publication of application:
**22.02.2012 Bulletin 2012/08**

(73) Proprietor: **BIO-FD&C Co., Ltd.**
**Namdong-gu, Incheon 405-849 (KR)**

(72) Inventors:
- **Jung, Dai Hyun**
  **Gwangju 506-304 (KR)**
- **Moh, Sang Hyun**
  **Yongin-si**
  **Gyeonggi-do 446-752 (KR)**
- **Lee, Jeong Hun**
  **Seoul 151-015 (KR)**
- **Kim, Su Jung**
  **Gwangju 506-304 (KR)**
- **Kim, Hyoung Shik**
  **Incheon 406-090 (KR)**
- **Seo, Hyo Hyun**
  **Yeonsu-gu**
  **Incheon 406-113 (KR)**
- **Lim, Chang Il**
  **Hwasun-gun**
  **Jeollanam-do 519-762 (KR)**
- **Cho, Moon Jin**
  **Bupyeong-gu**
  **Incheon 403-132 (KR)**
- **Sung, Ji Yeon**
  **Seo-gu**
  **Gwangju 502-865 (KR)**
- **Min, Ji Aee**
  **Bupyeong-gu**
  **Incheon 403-010 (KR)**

(74) Representative: **Jansen, Cornelis Marinus et al**
**VEREENIGDE**
**Johan de Wittlaan 7**
**2517 JR Den Haag (NL)**

(56) References cited:
**WO-A-83/03968 WO-A-2009/012376**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 420 510 B1

## Description

[Technical Field]

The present invention relates to nicotinoyl peptide derivatives and a cosmetic composition comprising the same. More particularly, the present invention relates to nicotinoyl peptide derivatives having an excellent anti-wrinkle effect through good collagen generation and anti-inflammatory activities, as well as little cytotoxicity, water-solubility, and good stability during long-term storage, and a cosmetic composition comprising the same.

[Background Art]

Although people have a desire to keep their youth as they grow old, they cannot stop the biological aging process. Therefore, many researchers have studied to estimate and prevent the skin aging previously.

The skin aging is generally divided into intrinsic aging and extrinsic aging. The intrinsic aging is an inherent degenerative process due to declining physiologic functions and capacities, and clinically characterized by decline of skin elasticity, rough skin, deep furrows, and pigmentation [*see*: Arch Dermatol., 130: 87-95, 1994, which is incorporated in its entirety here by reference]. The extrinsic aging is a distinctive declination process caused by external factors such as reactive oxygen species (ROS) and stress, which generates rough wrinkles in the skin surface and significantly decreases skin elasticity.

The causes of the skin aging usually include declining cell functions due to genetic damage, cell damage by free radicals, accumulating toxic materials, abnormal immune system, etc. Among them, the free radicals are known to be a main cause of the skin aging. In particular, reactive oxygen species among the free radicals come out of cells and induce inflammation in the surrounding tissue to stimulate the aging of the cells.

When cells or tissues are damaged, the inflammation occurs to minimize the response and recover the damaged regions. The causes of the inflammation include physical factors such as wound, bum, frostbite and radioactivity, chemical factors such as acid and base, and immunologic factors such as antibody reaction. Also, the inflammation is caused by blood vessel or hormone imbalance.

A variety of compounds have been developed to control the wrinkles and undesired forms of skin surface tissues. It is reported that vitamin B3 compounds, particularly niacinamide plays an important role in controlling wrinkles or uneven skin conditions due to age or damage by UV radiation, etc. However, the niacinamide is known to show an anti-wrinkle effect by stimulating collagen generation at high concentration.

Therefore, there has been a need to develop a new compound to have an anti-aging effect by stimulating collagen generation even at low concentration, as well as by having anti-inflammatory activity.

WO-A-2009012376 teaches the use of several compounds all comprised among the delta opioid receptor agonists, including enkephalin, as cosmetic agents. This document teaches no conjugates of these peptides with nicotinic acid for cosmetic use.

[Disclosure]

[Technical Problem]

The present inventors have conducted research to develop a compound that has superior collagen generation and anti-inflammatory activities, and found that the nicotinoyl peptide derivatives according to the present invention, wherein neuropeptides are connected to nicotinic acid, have excellent collagen generation and anti-inflammatory activities with low skin stimulation.

An object of the present invention is, therefore, to provide nicotinoyl peptide derivatives having an excellent anti-wrinkle effect through good collagen generation and anti-inflammatory activities, as well as little cytotoxicity, water-solubility, and good stability during long-term storage.

Another object of the present invention is to provide an anti-aging cosmetic composition comprising the nicotinoyl peptide derivative.

[Technical Solution]

The present invention relates to a peptide derivative of the following formula (I), which can be used as an ingredient for functional cosmetics.

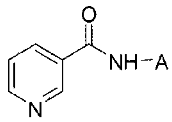

(I)

wherein,
A is a neuropeptide consisting of 5 to 31 amino acid residues comprising a peptide fragment selected from the group consisting of Tyr-Gly-Gly-Phe-Met and Tyr-Gly-Gly-Phe-Leu.

In one embodiment of the present invention, A is particularly:

Tyr-Gly-Gly-Phe-Met (SEQ ID No:1),
Tyr-Gly-Gly-Phe-Leu (SEQ ID No:2),
Tyr-Gly-Gly-Phe-Leu-Arg-Lys-Tyr-Pro (SEQ ID No:3),
Tyr-Gly-Gly-Phe-Leu-Arg-Lys-Tyr-Pro-Lys (SEQ ID No:4),
Tyr-Gly-Gly-Phe-Leu-Gly-His-Lys (SEQ ID No:5),
Tyr-Gly-Gly-Phe-Leu-Lys-Thr-Thr-Lys-Ser (SEQ ID No:6),
Tyr-Gly-Gly-Phe-Leu-Glu-Glu-Met-Gln-Arg-Arg (SEQ ID No:7),
Tyr-Gly-Gly-Phe-Leu-Arg-Arg-Ile-Arg-Pro-Lys-Leu-Lys (SEQ ID No:8),
Tyr-Gly-Gly-Phe-Met-Thr-Ser-Glu-Lys-Ser-Gln-Thr-Pro-Leu-Val-Thr (SEQ ID No:9),
Tyr-Gly-Gly-Phe-Met-Thr-Ser-Glu-Lys-Ser-Gln-Thr-Pro-Leu-Val-Thr-Leu (SEQ ID No: 10), or
Tyr-Gly-Gly-Phe-Met-Thr-Ser-Glu-Lys-Ser-Gln-Thr-Pro-Leu-Val-Thr-Leu-Phe-Lys-Asn-Ala-Ile-Ile-Lys-Asn-Ala-Tyr-Lys-Lys-Gly-Glu (SEQ ID No: 11),

more particularly:

Tyr-Gly-Gly-Phe-Met (SEQ ID No:1),
Tyr-Gly-Gly-Phe-Leu (SEQ ID No:2),
Tyr-Gly-Gly-Phe-Leu-Arg-Lys-Tyr-Pro (SEQ ID No:3), or
Tyr-Gly-Gly-Phe-Leu-Arg-Lys-Tyr-Pro-Lys (SEQ ID No:4).

In this description, common rules for peptide nomenclature based on three or one letter amino acid codes apply unless mentioned otherwise. In other words, the central portion of the amino acid structure is represented by the three letter code (e.g., Ala, Lys) or one letter code (e.g., A, K), and L-configuration is assumed unless D-configuration is specifically indicated by "D-" followed by the three letter code (e.g., D-Ala, D-Lys).

In one embodiment, the amino acid residues of the peptide can be natural or non-natural amino acid residues.

The nicotinoyl peptide derivative according to the present invention can be prepared by synthesizing a desired peptide and coupling the peptide with nicotinic acid.

In one embodiment, the desired peptide can be prepared by extracting an *in vivo* protein and treating the protein with a protease, by using a genetic recombination and protein expression system, or particularly by a chemical synthetic method using a peptide synthesizer.

For example, the peptide derivative according to the present invention can be prepared by a process comprising the steps of:

(i) obtaining a $NH_2$-protected peptide-resin by a well-known solid phase peptide synthesis (SPPS);
(ii) reacting the obtained $NH_2$-protected peptide-resin with nicotinic acid; and
(iii) removing the resin.

In the case where a functional group is located at a side chain of an amino acid residue of the desired peptide, the functional group-protected amino acid can be used to synthesize the peptide in step (i), and the protecting group attached to the functional group can be removed in step (iii).

An exemplary process for preparing the peptide derivative according to the present invention is simply illustrated in the following Reaction Scheme 1.

[Reaction Scheme 1]

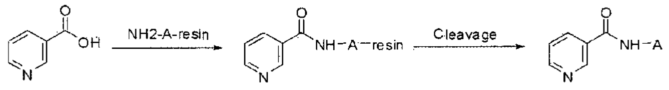


The present invention further relates to a cosmetic composition, particularly an anti-aging cosmetic composition, comprising the peptide derivative of the above formula (I) according to the present invention.

The peptide derivative according to the present invention has an excellent anti-wrinkle effect through good collagen generation and anti-inflammatory activities. Also, the peptide derivative has little cytotoxicity, water-solubility, and good stability during long-term storage. Therefore, the peptide derivative can be effectively used for preparing an anti-aging cosmetic composition.

In one embodiment, the cosmetic composition of the present invention can contain 0.005 to 1 wt% of the nicotinoyl peptide derivative according to the present invention.

In one embodiment, the cosmetic composition of the present invention can include cosmetic ingredients usually used in the art, for example, common adjuvants such as stabilizer, solubilizer, vitamin, colorant and perfumery, and carriers, in addition to the peptide derivative according to the present invention.

In one embodiment, the cosmetic composition of the present invention can be formulated as any form usually used in the art, for example, solution, suspension, emulsion, paste, gel, cream, powder, spray, etc.

In the case where the cosmetic composition is formulated as paste, cream or gel, the examples of the carriers include animal oil, vegetable oil, wax, paraffin, starch, tragacanth, cellulose derivative, polyethylene glycol, silicon, bentonite, silica, talc, zinc oxide, etc.

In the case where the cosmetic composition is formulated as powder or spray, the examples of the carriers include lactose, talc, silica, aluminum hydroxide, calcium silicate, polyamide powder, etc. In particular, in the case where the form is spray, the cosmetic composition can further include propellant such as chlorofluorohydrocarbon, propane/butane and dimethylether.

In the case where the cosmetic composition is formulated as solution or emulsion, the examples of the carriers include solvent, solubilizer and emulsifier such as water, ethanol, isopropanol, ethyl carbonate, ethyl acetate, benzyl alcohol, benzyl benzoate, propylene glycol, 1,3-butylglycol oil, glycerol fatty acid ester, polyethylene glycol, sorbitan fatty acid ester, etc.

In the case where the cosmetic composition is formulated as suspension, the examples of the carriers include liquid diluent such as water, ethanol and propylene glycol, suspending agent such as ethoxylated isostearyl alcohol, polyoxyethylene sorbitol ester and polyoxyethylene sorbitan ester, microcrystalline cellulose, aluminum methahydroxide, bentonite, agar, tragacanth, etc.

In one embodiment, the cosmetic composition of the present invention can be applied to cosmetics such as skin lotion, cream, essence, pack, foundation, coloring cosmetics, sun block cream, two-way cake, face powder, compact, makeup base, skin cover, eye shadow, lip stick, lip gloss, lip fix, eyebrow pencil and tonic, and cleaners such as shampoo and soap.

[Advantageous Effects]

The nicotinoyl peptide derivatives according to the present invention, wherein neuropeptides are connected to nicotinic acid, have superior collagen generation and anti-inflammatory activities to show an excellent anti-wrinkle effect. Also, the peptide derivative has little cytotoxicity, water-solubility, and good stability during long-term storage. Therefore, the peptide derivative can be effectively used for preparing an anti-aging cosmetic composition as a cosmetic ingredient.

[Best Mode]

The present invention is further illustrated by the following examples, which are not to be construed to limit the scope of the invention.

**Example 1: Preparation of nicotinoyl-pentapeptide (tyrosyl-glycyl-glycyl-phenylalanyl-methionine (SEQ ID No: 1))**

1.1: Synthesis of $NH_2$-protected peptide-resin

The desired peptide was synthesized by the well-known solid phase peptide synthesis (SPPS) using 9-fluorenylmethoxycarbonyl (Fmoc) as an amino acid protecting group, and the amino acid residues thereof were extended

using N-hydroxybenzotriazole (HOBt) and N,N'-dicyclohexylcarbodiimide (DCC) as an activating agent [*see*: Wang C. Chan, Perter D. White, 'Fmoc solid phase peptide synthesis', Oxford, which is incorporated in its entirety here by reference].

1.2: Synthesis of nicotinoyl-pentapeptide

The $NH_2$-protected peptide (tyrosyl-glycyl-glycyl-phenylalanyl-methionine)-resin obtained as above was reacted with 20% piperidine/N-methyl-2-pyrrolidone (NMP) solution to remove the Fmoc attached to the amino group, washed with NMP and dichloromethane (DCM), and subjected to a coupling reaction with 5 equivalents of nicotinic acid at room temperature overnight. After the reaction was completed, the resin was washed several times with NMP and DCM, and dried.

The dried nicotinoyl-pentapeptide-resin was reacted with a mixture of trifluoroacetic acid : phenol : thioanisole : water : ethanedithiol (82.5 : 5 : 5 : 5 : 2.5 (v/v)) at room temperature for 2 to 3 hours to remove the functional group protecting group, t-butyloxycarbonyl (Boc), and separate the nicotinoyl-pentapeptide from the resin. Then, the peptide was precipitated with cold diethylether.

The obtained nicotinoyl-pentapeptide was purified by reverse phase high performance liquid chromatography (column: Gemini, C18 110A 250x21.2 mm) using water and acetonitrile containing 0.1% trifluoroacetic acid as an eluent. Yield: 65%

**Example 2: Preparation of nicotinoyl-pentapeptide (tyrosyl-glycyl-glycyl-Phenylalanyl-leucine (SEO ID No:2))**

2.1: Synthesis of $NH_2$-protected peptide-resin

The $NH_2$-protected peptide (tyrosyl-glycyl-glycyl-phenylalanyl-leucine)-resin was synthesized in accordance with the process described in Example 1.

2.2: Synthesis of nicotinoyl-pentapeptide

The $NH_2$-protected peptide (tyrosyl-glycyl-glycyl-phenylalanyl-leucine)-resin obtained as above was reacted with 20% piperidine/NMP solution to remove the Fmoc attached to the amino group, washed with NMP and DCM, and subjected to a coupling reaction with 5 equivalents of nicotinic acid at room temperature overnight. After the reaction was completed, the resin was washed several times with NMP and DCM, and dried.

The dried nicotinoyl-pentapeptide-resin was reacted with a mixture of trifluoroacetic acid : phenol : thioanisole : water : ethanedithiol (82.5 : 5 : 5 : 5 : 2.5 (v/v)) at room temperature for 2 to 3 hours to remove the functional group protecting group, t-butyloxycarbonyl (Boc), and separate the nicotinoyl-pentapeptide from the resin. Then, the peptide was precipitated with cold diethylether.

The obtained nicotinoyl-pentapeptide was purified by reverse phase high performance liquid chromatography (column: Gemini, C18 110A 250x21.2 mm) using water and acetonitrile containing 0.1% trifluoroacetic acid as an eluent. Yield: 62 %

**Example 3: Preparation of nicotinoyl-nonapeptide (tyrosyl-glycyl-glycyl-phenylalanyl-leucyl-arginyl-lysyl-tyrosyl-proline (SEQ ID No:3))**

2.1: Synthesis of $NH_2$-protected peptide-resin

The $NH_2$-protected peptide (tyrosyl-glycyl-glycyl-phenylalanyl-leucyl-arginyl-lysyl-tyrosyl-proline)-resin was synthesized in accordance with the process described in Example 1.

2.2: Synthesis of nicotinoyl-nonapeptide

The $NH_2$-protected peptide (tyrosyl-glycyl-glycyl-phenylalanyl-leucyl-arginyl-lysyl-tyrosyl-proline)-resin obtained as above was reacted with 20% piperidine/NMP solution to remove the Fmoc attached to the amino group, washed with NMP and DCM, and subjected to a coupling reaction with 5 equivalents of nicotinic acid at room temperature overnight. After the reaction was completed, the resin was washed several times with NMP and DCM, and dried.

The dried nicotinoyl-nonapeptide-resin was reacted with a mixture of trifluoroacetic acid : phenol : thioanisole : water : ethanedithiol (82.5 : 5 : 5 : 5 : 2.5 (v/v)) at room temperature for 2 to 3 hours to remove the functional group protecting groups, t-butyloxycarbonyl (Boc), t-buty (t-Bu) and 2,2,4,6,7-pentamethyldihydrobenzofuran-5-sulfonyl (Pbf), and separate the nicotinoyl-nonapeptide from the resin. Then, the peptide was precipitated with cold diethylether.

The obtained nicotinoyl-nonapeptide was purified by reverse phase high performance liquid chromatography (column: Gemini, C18 110A 250×21.2 mm) using water and acetonitrile containing 0.1% trifluoroacetic acid as an eluent. Yield: 57 %

**Example 4: Preparation of nicotinoyl-decapeptide (tyrosyl-glycyl-glycyl-phenylalanyl-leucyl-arginyl-lysyl-tyrosyl-prolyl-lycine (SEQ ID No:4))**

2.1: Synthesis of $NH_2$-protected peptide-resin

The $NH_2$-protected peptide (tyrosyl-glycyl-glycyl-phenylalanyl-leucyl-arginyl-lysyl-tyrosyl-prolyl-lycine)-resin was synthesized in accordance with the process described in Example 1.

2.2: Synthesis of nicotinoyl-decapeptide

The $NH_2$-protected peptide (tyrosyl-glycyl-glycyl-phenylalanyl-leucyl-arginyl-lysyl-tyrosyl-prolyl-lycine)-resin obtained as above was reacted with 20% piperidine/NMP solution to remove the Fmoc attached to the amino group, washed with NMP and DCM, and subjected to a coupling reaction with 5 equivalents of nicotinic acid at room temperature overnight. After the reaction was completed, the resin was washed several times with NMP and DCM, and dried.

The dried nicotinoyl-decapeptide-resin was reacted with a mixture of trifluoroacetic acid : phenol : thioanisole : water : ethanedithiol (82.5 : 5 : 5 : 5 : 2.5 (v/v)) at room temperature for 2 to 3 hours to remove the functional group protecting groups, t-butyloxycarbonyl (Boc), t-buty (t-Bu) and 2,2,4,6,7-pentamethyldihydrobenzofuran-5-sulfonyl (Pbf), and separate the nicotinoyl-decapeptide from the resin. Then, the peptide was precipitated with cold diethylether.

The obtained nicotinoyl-decapeptide was purified by reverse phase high performance liquid chromatography (column: Gemini, C18 110A 250×21.2 mm) using water and acetonitrile containing 0.1% trifluoroacetic acid as an eluent. Yield: 55 %

**Comparative Example 1: Preparation of nicotinoyl-tripeptide (γ-glutamyl-cysteinyl-glycine (SEO ID No:13))**

The target compound was prepared in accordance with the process described in Example 1. Yield: 41%

**Comparative Example 2: Preparation of nicotinoyl-pentapeptide (lysyl-threonyl-threonyl-lysyl-serine (SEQ ID No:12))**

The target compound was prepared in accordance with the process described in Example 1. Yield: 53%

**Experimental Example 1: Cytotoxicity test**

Human fibroblasts were seeded to a 24-well plate in $1\times10^4$ cells/mℓ and cultured in Dulbecco's modified Eagle's medium (DMEM) supplemented with 10% fetal bovine serum (FBS) (BRL, USA) for 48 hours.

After 25 to 30% of the surface area of the culture vessel was cultured, the culture solution was replaced with DMEM without FBS containing 100 μ M of the nicotinoyl peptide derivative respectively obtained in Examples 1 to 4 and Comparative Examples 1 and 2, and further cultured for 24 hours.

50 μl of 3-(4,5-dimethylthiazol-2-yl)-2,5-diphenyltetrazolium bromide (MTT, Sigma M5655, USA) solution (2.5 mg/mℓ) was added to the culture solution, followed by further culturing for 3 hours. Then, the culture solution was centrifuged to remove a supernatant, and 200 μl of dimethylsulfoxide (DMSO, Sigma D2650, USA) was added to each well and vortexed for 20 minutes to dissolve formazan crystals formed. 100 μl of the resulting solution was added to a 96-well plate and its absorbance was measured at 570 nm with enzyme-linked immunosorbent assay (ELISA).

The cell growth effect (cytotoxicity) was calculated according to the following Formula 1, based on the absorbance of the control using purified water, and the results (%) are shown in Table 1.

[Formula 1]

$$\text{Cell growth effect} = \frac{\text{Absorbance of peptide treated group}}{\text{Absorbance of control}} \times 100$$

[Table 1]

| Classification | Sample | Molecular Weight | Cell Growth Effect (%) |
|---|---|---|---|
| Control | Purified water | - | 100 |
| Example 1 | Nicotinoyl-YGGFM | 678.7 | 102.1 |
| Example 2 | Nicotinoyl-YGGFL | 660.7 | 101.3 |
| Example 3 | Nicotinoyl-YGGFLRKYP | 1205.3 | 99.1 |
| Example 4 | Nicotinoyl-YGGFLRKYPK | 1333.5 | 98.8 |
| Comparative Example 1 | Nicotinoyl-γ-ECG | 412.4 | 98.3 |
| Comparative Example 2 | Nicotinoyl-KTTKS | 668.7 | 97.6 |
| | Nicotinic acid | 123.1 | 102.3 |
| | Nicotinamide | 122.1 | 103.2 |

As shown in Table 1, the nicotinoyl peptide derivatives obtained in Examples 1 to 4 had little cytotoxicity at a concentration of 100 μM to be safe.

**Experimental Example 2: Collagen generation activity test**

Human fibroblasts (primary cell line, CCD-986sk) were seeded to a plate and cultured in Dulbecco's modified Eagle's medium (DMEM) supplemented with penicillin (100 IU/ml), streptomycin (100μg/ml) and 10% fetal bovine serum (FBS) at 37 °C with 5% $CO_2$.

The cultured cells were divided to a 48-well plate in $5\times10^4$ cells/well and cultured for 24 hours. Then, the cultured cells were washed with 10% phosphate buffered saline (PBS), treated with 100 μ M of the nicotinoyl peptide derivative respectively obtained in Examples 1 to 4 and Comparative Examples 1 and 2, and further cultured for 24 hours. The amount of collagen generated in the culture solution was measured with procollagen type I C-peptide (PIP) (Takara, MK101) ELISA.

After 100 μℓ of antibody-PoD conjugate solution was added to each well, 20 μℓ of the culture solution diluted to 1/5 and standard solution were added and cultured at 37 °C for 3 hours. Then, each well was washed four times with 400 μℓ of PBS. 100 μℓ of color coupler was added and cultured at room temperature for 15 minutes. Then, 100 μℓ of IN sulfuric acid was added and the absorbance was measured at 450 nm with a ELISA reader.

The collagen generation activity was calculated according to the following Formula 2, based on the absorbance of the control using purified water, and the results (%) are shown in Table 2.

[Formula 2]

$$\text{Collagen generation activity} = \frac{\text{Absorbance of peptide treated group}}{\text{Absorbance of control}} \times 100$$

[Table 2]

| Classification | Sample | Molecular Weight | Collagen Generation Activity (%) |
|---|---|---|---|
| Control | Purified water | - | 100 |
| Example 1 | Nicotinoyl-YGGFM | 678.7 | 123.3 |
| Example 2 | Nicotinoyl-YGGFL | 660.7 | 119.5 |
| Example 3 | Nicotinoyl-YGGFLRKYP | 1205.3 | 122.8 |
| Example 4 | Nicotinayl-YGGFLRKYPK | 1333.5 | 123.0 |
| Comparative Example 1 | Nicotinoyl-γ-ECG | 412.4 | 99.5 |
| Comparative Example 2 | Nicotinoyl-KTTKS | 668.7 | 100.5 |
| | Nicotinic acid | 123.1 | 105.2 |

(continued)

| Classification | Sample | Molecular Weight | Collagen Generation Activity (%) |
|---|---|---|---|
| | Nicotinamide | 122.1 | 108.8 |

As shown in Table 2, the nicotinoyl peptide derivatives obtained in Examples 1 to 4 had significantly increased collagen generation activity, as compared with nicotinic acid and nicotinamide, and had better collagen generation activity than the nicotinoyl peptide derivative of Comparative Example 1, wherein a glutathione-like peptide was connected to nicotinic acid, and the nicotinoyl peptide derivative of Comparative Example 2, wherein a collagen derived peptide was connected to nicotinic acid.

**Experimental Example 3: Anti-inflammatory activity test (iNOS activity inhibition test)**

Raw264.7 cells (murine macrophage cell line) were suspended in 10% fetal bovine serum-Dulbecco's modified Eagle's medium (FBS-DMEM) without iNOS (GIBCO) in $L\times10^5$ cells/mℓ and seed to a 24-well plate.

After overnight incubation, the cultured cells were treated with 100 μ M of the nicotinoyl peptide derivative respectively obtained in Examples 1 to 4 and Comparative Examples 1 and 2, and cultured for 18 hours. Then, 1 μg/μℓ of lipopolysaccharide (LPS, Sigma) was added and cultured for 24 hours. Afterwards, 100μℓ of the obtained supernatant was added to a 96-well plate and the same amount of Griess reagent (Sigma) was added, followed by light vortexing at room temperature for 10 minutes. The absorbance was measured at 570 nm.

The calibration curve was prepared using sodium nitrite as a standard, and the amount of nitric oxide produced in the culture solution was calculated. The yield of each sample was calculated, based on the amount of nitric oxide produced in the group treated with LPS only, and the results (%) are shown in Table 3.

[Table 3]

| Classification | Sample | Molecular Weight | Yields (%) |
|---|---|---|---|
| Control | LPS | - | 100 |
| | LPS non-treatment (DMEM) | - | 30.5 |
| Example 1 | LPS + Nicotinoyl-YGGFM | 678.7 | 78.5 |
| Example 2 | LPS + Nicotinoyl-YGGFL | 660.7 | 80.9 |
| Example 3 | LPS + Nicotinoyl-YGGFLRKYP | 1205.3 | 75.1 |
| Example 4 | LPS + Nicotinoyl-YGGFLRKYPK | 1333.5 | 77.8 |
| Comparative Example 1 | LPS + Nicotinoyl-γ-ECG | 412.4 | 94.5 |
| Comparative Example 2 | LPS + Nicotinoyl-KTTKS | 668.7 | 95.1 |
| | LPS + Nicotinic acid | 123.1 | 98.8 |
| | LPS + Nicotinamide | 122.1 | 96.1 |

As shown in Table 3, the nicotinoyl peptide derivatives obtained in Examples 1 to 4 inhibited the iNOS activity. In particular, the nicotinoyl peptide derivatives according to the present invention significantly inhibited the iNOS activity, as compared with nicotinic acid, nicotinamide, the nicotinoyl peptide derivative of Comparative Example 1, wherein a glutathione-like peptide was connected to nicotinic acid, and the nicotinoyl peptide derivative of Comparative Example 2, wherein a collagen derived peptide was connected to nicotinic acid.

**Formulation Example 1: Preparation of skin tonic comprising nicotinoyl peptide derivative**

A skin tonic comprising the nicotinoyl peptide derivative obtained in Example 1 was prepared according to the composition and contents of Table 4.

Particularly, ingredient Nos. 2, 3, 4 and 8 were added to ingredient No. 11 sequentially and stirred to give a solution (Solution 1). Meanwhile, ingredient No. 5 was heated to 60 °C to melt, and ingredient No. 10 was added thereto to give a solution (Solution 2). Then, Solution 2 was added to Solution 1, and ingredient Nos. 1, 6, 7 and 9 were added. The mixture was stirred and matured to give the skin tonic.

[Table 4]

| No. | Ingredient | Content (wt%) |
|---|---|---|
| 1 | Nicotinoyl peptide derivative obtained in Example 1 | 0.01 |
| 2 | Glycerine | 3.0 |
| 3 | Butylene glycol | 2.0 |
| 4 | Propylene glycol | 2.0 |
| 5 | Polyoxyethylene hardened castor oil | 1.5 |
| 6 | Ethanol | 8.5 |
| 7 | Triethanolamine | 0.1 |
| 8 | Preservative | Trace |
| 9 | Colorant | Trace |
| 10 | Perfumery | Trace |
| 11 | Purified water | Balance |

**Formulation Example 2: Preparation of lotion comprising nicotinoyl peptide derivative**

A lotion comprising the nicotinoyl peptide derivative obtained in Example 1 was prepared according to the composition and contents of Table 5.

Particularly, ingredient Nos. 10, 11, 13 and 16 were mixed with stirring and heated to 80-85 °C. The mixture was added to a production device and emulsified. Then, ingredient Nos. 2, 3, 4, 5, 6, 7, 8, 9 and 12, which were heated to 80-85 °C to melt, were added to the production device and emulsified. After the emulsification was completed, the resulting emulsion was cooled to 50 °C with stirring. Then, ingredient No. 15 was added and cooled to 45 °C, and ingredient No. 14 was added. Afterwards, ingredient No. 1 was added at 35 °C, and the mixture was cooled to 25 °C and matured to give the lotion.

[Table 5]

| No. | Ingredient | Content (wt%) |
|---|---|---|
| 1 | Nicotinoyl peptide derivative obtained in Example 1 | 0.01 |
| 2 | Wax | 1.0 |
| 3 | Polysolvate 60 | 2.0 |
| 4 | Sorbitan sesquioleate | 0.5 |
| 5 | Fluid paraffin | 9.5 |
| 6 | Sorbitan stearate | 1.0 |
| 7 | Lipophilic glycerine monostearate | 0.2 |
| 8 | Stearic acid | 1.5 |
| 9 | Glycerine stearate | 1.0 |
| 10 | Propylene glycol | 2.5 |
| 11 | Carboxy polymer | 0.1 |
| 12 | Triethanolamine | 0.2 |
| 13 | Preservative | Trace |
| 14 | Colorant | Trace |
| 15 | Perfumery | Trace |
| 16 | Purified water | Balance |

**Claims**

**1.** A peptide derivative of the following formula (I):

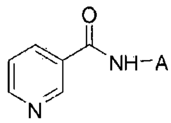

(I)

wherein,
A is a neuropeptide consisting of 5 to 31 amino acid residues comprising a peptide fragment selected from the group consisting of Tyr-Gly-Gly-Phe-Met and Tyr-Gly-Gly-Phe-Leu.

**2.** The peptide derivative of Claim 1, wherein A is:

Tyr-Gly-Gly-Phe-Met (SEQ ID No:1),
Tyr-Gly-Gly-Phe-Leu (SEQ ID No:2),
Tyr-Gly-Gly-Phe-Leu-Arg-Lys-Tyr-Pro (SEQ ID No:3),
Tyr-Gly-Gly-Phe-Leu-Arg-Lys-Tyr-Pro-Lys (SEQ ID No:4),
Tyr-Gly-Gly-Phe-Leu-Gly-His-Lys (SEQ ID No:5),
Tyr-Gly-Gly-Phe-Leu-Lys-Thr-Thr-Lys-Ser (SEQ ID No:6),
Tyr-Gly-Gly-Phe-Leu-Glu-Glu-Met-Gln-Arg-Arg (SEQ ID No:7),
Tyr-Gly-Gly-Phe-Leu-Arg-Arg-Ile-Arg-Pro-Lys-Leu-Lys (SEQ ID No:8),
Tyr-Gly-Gly-Phe-Met-Thr-Ser-Glu-Lys-Ser-Gln-Thr-Pro-Leu-Val-Thr (SEQ ID No:9),
Tyr-Gly-Gly-Phe-Met-Thr-Ser-Glu-Lys-Ser-Gln-Thr-Pro-Leu-Val-Thr-Leu (SEQ ID No: 10), or
Tyr-Gly-Gly-Phe-Met-Thr-Ser-Glu-Lys-Ser-Gln-Thr-Pro-Leu-Val-Thr-Leu-Phe-Lys-Asn-Ala-Ile-Ile-Lys-Asn-Ala-Tyr-Lys-Lys-Gly-Glu (SEQ ID No:11).

**3.** The peptide derivative of Claim 2, wherein A is:

Tyr-Gly-Gly-Phe-Met (SEQ ID No:1),
Tyr-Gly-Gly-Phe-Leu (SEQ ID No:2),
Tyr-Gly-Gly-Phe-Leu-Arg-Lys-Tyr-Pro (SEQ ID No:3), or
Tyr-Gly-Gly-Phe-Leu-Arg-Lys-Tyr-Pro-Lys (SEQ ID No:4).

**4.** A cosmetic composition comprising the peptide derivative of any one of Claims 1 to 3.

**5.** Use of the cosmetic composition of Claim 4 for skin anti-aging.

**Patentansprüche**

**1.** Peptidderivat der folgenden Formel (I):

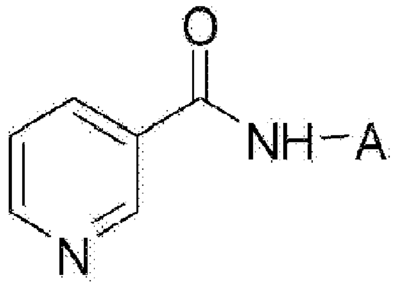

(I)

wobei,
A ein Neuropeptid bestehend aus 5 bis 31 Aminosäureresten ist, umfassend ein Peptidfragment ausgewählt aus der Gruppe bestehend aus Tyr-Gly-Gly-Phe-Met und Tyr-Gly-Gly-Phe-Leu.

**2.** Das Peptidderivat nach Anspruch 1, wobei A ist:

Tyr-Gly-Gly-Phe-Met (SEQ ID No: 1),
Tyr-Gly-Gly-Phe-Leu (SEQ ID No: 2),
Tyr-Gly-Gly-Phe-Leu-Arg-Lys-Tyr-Pro (SEQ ID No: 3),
Tyr-Gly-Gly-Phe-Leu-Arg-Lys-Tyr-Pro-Lys (SEQ ID No: 4),
Tyr-Gly-Gly-Phe-Leu-Gly-His-Lys (SEQ ID No: 5),
Tyr-Gly-Gly-Phe-Leu-Lys-Thr-Thr-Lys-Ser (SEQ ID No: 6),
Tyr-Gly-Gly-Phe-Leu-Glu-Glu-Met-Gln-Arg-Arg (SEQ ID No: 7),
Tyr-Gly-Gly-Phe-Leu-Arg-Arg-Ile-Arg-Pro-Lys-Leu-Lys (SEQ ID No.: 8),
Tyr-Gly-Gly-Phe-Met-Thr-Ser-Glu-Lys-Ser-Gln-Thr-Pro-Leu-Val-Thr (SEQ ID No: 9),
Tyr-Gly-Gly-Phe-Met-Thr-Ser-Glu-Lys-Ser-Gln-Thr-Pro-Leu-Val-Thr-Leu (SEQ ID No: 10) oder
Tyr-Gly-Gly-Phe-Met-Thr-Ser-Glu-Lys-Ser-Gln-Thr-Pro-Leu-Val-Thr-Leu-Phe-Lys-Asn-Ala-Ile-Ile-Lys-Asn-Ala-Tyr-Lys-Lys-Gly-Glu (SEQ ID No: 11).

**3.** Das Peptidderivat nach Anspruch 2, wobei A ist:

Tyr-Gly-Gly-Phe-Met (SEQ ID No: 1),
Tyr-Gly-Gly-Phe-Leu (SEQ ID No: 2),
Tyr-Gly-Gly-Phe-Leu-Arg-Lys-Tyr-Pro (SEQ ID No: 3), oder
Tyr-Gly-Gly-Phe-Leu-Arg-Lys-Tyr-Pro-Lys (SEQ ID No: 4).

**4.** Kosmetische Zusammensetzung umfassend das Peptidderivat nach einem der Ansprüche 1 bis 3.

**5.** Verwendung der kosmetischen Zusammensetzung nach Anspruch 4 gegen Hautalterung.

## Revendications

**1.** Dérivé peptidique de formule (I) suivante :

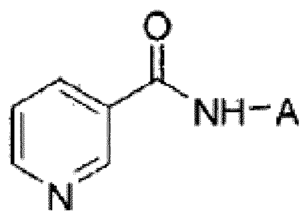

(I)

où,
A est un neuropeptide consistant en 5 à 31 résidus d’aminoacide comprenant un fragment peptidique choisi dans

le groupe consistant en Tyr-Gly-Gly-Phe-Met et Tyr-Gly-Gly-Phe-Leu.

**2.** Dérivé peptidique selon la revendication 1, où A est :

Tyr-Gly-Gly-Phe-Met (SEQ ID No:1),
Tyr-Gly-Gly-Phe-Leu (SEQ ID No:2).
Tyr-Cly-Gly-Phe-Leu-Arg-Lys-Tyr-Pro (SEQ ID No:3).
Tyr-Gly-Gly-Phe-Leu-Arg-Lys-Tyr-Pro-Lys (SEQ ID No:4),
Tyr-Gly-Gly-Phe-Leu-Gly-His-Lys (SEQ ID No:5),
Tyr-Gly-Gly-Phe-Leu-Lys-Thr-Thr-Lys-Ser (SEQ ID No:6).
Tyr-Gly-Gly-Phe-Leu-Glu-Glu-Met-Gln-Arg-Arg (SEQ ID No:7),
Tyr-Gly-Gly-Phe-Leu-Arg-Arg-Ile-Arg-Pro-Lys-Leu-Lys (SEQ ID No:8),
Tyr-Gly-Gly-Phe-Met-Thr-Ser-Glu-Lys-Ser-Gln-Thr-Pro-Leu-Val-Thr (SEQ ID No:9),
Tyr-Gly-Gly-Phe-Met-Thr-Ser-Glu-Lys-Ser-Gln-Thr-Pro-Leu-Val-Leu (SEQ ID No: 10), ou
Tyr-Gly-Gly-Phe-Met-Thr-Ser-Glu-Lys-Ser-Gln-Thr-Pro-Leu-Val-Thr-Leu-Phe-Lys-Asn-Ala-Ile-Lys-Asn-Ala-Tyr-Lys-Gly-Glu (SEQ ID No:11).

**3.** Dérivé peptidique selon la revendication 2, où A est :

Tyr-Gly-Gly-Phe-Met (SEQ ID No:1),
Tyr-Gly-Gly-Phe-Leu (SEQ ID No:2),
Tyr-Gly-Gly-Phe-Leu-Arg-Lys-Tyr-Pro (SEQ ID No:3), ou
Tyr-Gly-Gly-Phe-Phe-Arg-Lys-Tyr-Pro-Lys (SEQ ID No:4).

**4.** Composition cosmétique comprenant le dérivé peptidique selon l'une quelconque des revendications 1 à 3.

**5.** Utilisation de la composition cosmétique selon la revendication 4 contre le vieillissement cutané.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009012376 A
- KR 1020100080687


### Non-patent literature cited in the description


- *Arch Dermatol.,* 1994, vol. 130, 87-95

<110> BIO-FD&C Co., Ltd.

<120> Nicotinoyl Peptide Derivatives and Cosmetic Composition Comprising the Same

<130> P95489EP00

<150> KR 10-2010-0080687
<151> 2010-08-20

<160> 13

<170> KopatentIn 2.0

<210> 1
<211> 5
<212> PRT
<213> Artificial Sequence

<220>
<223> Neuropeptide

<400> 1

```
Tyr Gly Gly Phe Met
 1               5
```

<210> 2
<211> 5
<212> PRT
<213> Artificial Sequence

<220>
<223> Neuropeptide

<400> 2

```
Tyr Gly Gly Phe Leu
 1               5
```

<210> 3
<211> 9
<212> PRT
<213> Artificial Sequence

<220>
<223> Neuropeptide

<400> 3

```
Tyr Gly Gly Phe Leu Arg Lys Tyr Pro
 1               5
```

<210> 4
<211> 10
<212> PRT
<213> Artificial Sequence

<220>

<223> Neuropeptide

<400> 4

Tyr Gly Gly Phe Leu Arg Lys Tyr Pro Lys
1 5 10

<210> 5
<211> 8
<212> PRT
<213> Artificial Sequence

<220>
<223> Neuropeptide

<400> 5

Tyr Gly Gly Phe Leu Gly His Lys
1 5

<210> 6
<211> 10
<212> PRT
<213> Artificial Sequence

<220>
<223> Neuropeptide

<400> 6

Tyr Gly Gly Phe Leu Lys Thr Thr Lys Ser
1 5 10

<210> 7
<211> 11
<212> PRT
<213> Artificial Sequence

<220>
<223> Neuropeptide

<400> 7

Tyr Gly Gly Phe Leu Glu Glu Met Gln Arg Arg
1 5 10

<210> 8
<211> 13
<212> PRT
<213> Artificial Sequence

<220>
<223> Neuropeptide

<400> 8

Tyr Gly Gly Phe Leu Arg Arg Ile Arg Pro Lys Leu Lys
1 5 10

<210> 9
<211> 16
<212> PRT
<213> Artificial Sequence

<220>
<223> Neuropeptide

<400> 9

Tyr Gly Gly Phe Met Thr Ser Glu Lys Ser Gln Thr Pro Leu Val Thr
1 5 10 15

<210> 10
<211> 17
<212> PRT
<213> Artificial Sequence

<220>
<223> Neuropeptide

<400> 10

Tyr Gly Gly Phe Met Thr Ser Glu Lys Ser Gln Thr Pro Leu Val Thr
1 5 10 15

Leu

<210> 11
<211> 31
<212> PRT
<213> Artificial Sequence

<220>
<223> Neuropeptide

<400> 11

Tyr Gly Gly Phe Met Thr Ser Glu Lys Ser Gln Thr Pro Leu Val Thr
1 5 10 15

Leu Phe Lys Asn Ala Ile Ile Lys Asn Ala Tyr Lys Lys Gly Glu
20 25 30

<210> 12
<211> 5
<212> PRT
<213> Artificial Sequence

<220>
<223> Collagen derived peptide

<400> 12

Lys Thr Thr Lys Ser
1 5

<210> 13
<211> 3
<212> PRT

<213> Artificial Sequence

<220>
<223> glutathion like peptide

<400> 13

Glu Cys Gly
1 5